# NEW EUROPEAN PATENT SPECIFICATION

(11) **EP 1 212 081 B2**
(45) Date of publication and mention of the opposition decision: **02.04.2014**
(45) Mention of the grant of the patent: 26.10.2005
(21) Application number: 00965898.0
(22) Date of filing: 25.08.2000
(51) Int. Cl.: A61K 31/4184, A61P 9/04, A61P 9/10, A61P 3/10

(54) **USE OF RAMIPRIL IN THE PREVENTION OF CONGESTIVE HEART FAILURE**
VERWENDUNG VON RAMIPRIL ZUR PROPHYLAXE VON KONGESTIVEM HERZVERSAGEN
UTILISATIONS DE RAMIPRIL POUR PREVENIR L'INSUFFISANCE CARDIAQUE GLOBALE

(30) Priority: 27.08.1999 SE 9903028
(43) Date of publication of application: 12.06.2002
(60) Divisional application: 04006330.7
(73) Proprietor: Sanofi-Aventis Deutschland GmbH, 65929 Frankfurt am Main (DE)
(72) Inventor: SCHOELKENS, Bernward, 65779 Kelkheim (DE); BENDER, Norbert, 65719 Hofheim (DE); RANGOONWALA, Badrudin, 65719 Hofheim (DE); DAGENAIS, Gilles, Quebec, J7A 3P8 (CA); GERSTEIN, Hertzel, Hamilton, Ontario L8S 4A8 (CA); LJUNGGREN, Anders, S-431 63 Mölndal (SE); YUSUF, Salim, Carlisle, Ontario L0R 1H2 (CA)
(86) International application number: PCT/EP2000/008341
(87) International publication number: WO 2001/015673

(56) References cited:
- EP-A- 0 241 201
- EP-A- 0 331 014
- EP-A- 0 426 066
- EP-A- 0 474 438
- EP-A- 0 540 209
- EP-A- 0 747 050
- EP-A- 0 795 327
- WO-A-00/02543
- WO-A-00/71751
- WO-A-01/15674
- WO-A-93/20839
- WO-A-96/24373
- WO-A-97/27745
- WO-A-97/49392
- WO-A-98/30216
- WO-A-99/20260
- WO-A-99/44590
- WO-A2-93/20839
- DE-A- 4 308 504
- DE-A- 19 913 528
- GB-A- 2 308 064
- US-A- 5 190 970
- US-A- 5 266 583
- US-A- 5 308 846
- US-A- 5 506 361
- DATABASE WPI Week 199944 Derwent Publications Ltd., London, GB; AN 1999-520858 XP002166044 MASUDA YOSHINOBU, HONDA YAYOI, MINATO HISAO: "Inhibitor of cerebral vasospasm" & JP 11 222439 A (DAINIPPON PHARM CO LTD), 17 August 1999 (1999-08-17)
- OGIKU N ET AL: "Prophylactic effect of imidapril on stroke in stroke -prone spontaneously hypertensive rats." STROKE, (1993 FEB) 24 (2) 245-52., XP000997781
- STIER C T JR ET AL: "ENALAPRIL PREVENTS STROKE AND KIDNEY DYSFUNCTION IN SALT-LOADED STROKE-PRONE SPONTANEOUSLY HYPERTENSIVE RATS" HYPERTENSION (DALLAS), vol. 13, no. 2, 1989, pages 115-121, XP000997775 ISSN: 0194-911X
- ROBERT MKW LEE, HONG WANG, JOHN S SMEDA: "Effects of perindopril on hypertension and stroke prevention in experimental animals" CAN. J. CARDIOL., vol. 10, no. Suppl D, November 1994 (1994-11), pages 33D-36D, XP000998206
- J.E.F. REYNOLDS: "Martindale The Extra Pharmacopoeia" 1996 , ROYAL PHARMACEUTICAL SOCIETY , LONDON XP002166043 page 863, column 2 -page 864, column 2 page 899, column 3 -page 900, column 1 page 940, column 3 -page 941, column 1
- EBERHARDT ROBERT T ET AL: "Angiotensin II receptor blockade: An innovative approach to cardiovascular pharmacotherapy." JOURNAL OF CLINICAL PHARMACOLOGY, vol. 33, no. 11, 1993, pages 1023-1038, XP001019614 ISSN: 0091-2700
- STIER CHARLES T JR ET AL: "Stroke prevention by losartan in stroke-prone spontaneously hypertensive rats." JOURNAL OF HYPERTENSION, vol. 11, no. SUPPL. 3, 1993, pages S37-S42, XP001019687 Meeting on Losartan: An Orally Active Angiotensin II Antagonist;St. Paul de Vence, France; June 26-27, 1992 ISSN: 0263-6352
- BLUMENTHAL MEL: "Treatment of congestive heart failure: Experience with fosinopril." AMERICAN JOURNAL OF HYPERTENSION, vol. 10, no. 10 PART 2, 1997, pages 289S-298S, XP001019673 ISSN: 0895-7061
- NAKAMURA FUMIAKI ET AL: "Chronic administration of angiotensin II receptor antagonist, TCV-116, in cardiomyopathic hamsters." AMERICAN JOURNAL OF PHYSIOLOGY, vol. 267, no. 6 PART 2, 1994, pages H2297-H2304, XP001019610 ISSN: 0002-9513
- K. PARFITT: "Martindale - The complete drug reference - Thirty-second Edition" 1999 , PHARMACEUTICAL PRESS , LONDON UK XP002176301 page 836, column 1 page 865, column 3 page 891, column 3 -page 892, column 1 page 899, column 1 page 951, column 3 page 960, column 2 -column 3
- BLUMENTHAL, M.: 'Treatment of Congestive Heart Failure Experience with Fosinopril' THE AMERICAN JOURNAL OF HYPERTENSION, LTD. vol. 10, no. 10, 1997, pages 289S - 298S
- VAN GILST, W.H. ET AL: 'Which Patient Benefits From Early Angiotensin-Converting Enzyme Inhibition AfterMyocardial Infarction?' JAAC vol. 28, no. 1, 1996, pages 114 - 121
- 'The HOPE (Heart Outcomes Prevention Evaluation) Study' CAN.J.CARDIOL. vol. 12, no. 2, February 1996, pages 127 - 137
- GEORGE, K.J.: 'Study findings "could prevent one million premature deaths each year"' MCMASTER UNIVERSITY NEWS RELEASE 11 May 1999,
- YUSUF, S. ET AL: 'Effects of an angiotensin-converting-enzyme inhibitor, ramipril, on cardiovascular events in high-risk patients' THE NEW ENGLAND JOURNAL OF MEDICINE vol. 342, no. 3, 20 January 2000, pages 145 - 153
- 'A Textbook of Cardiovascular Medicine, 7th Edition' BRAUNWALD'S HEART DISEASE ELSEVIER SAUNDERS, page 495
- 'The Sixth Report oh the Joint National Comittee on Prevention, Detection, Evaluation, and Treatment of high Blood Pressure' NIH PUBLICATION vol. 98-4080, November 1997,

## Description

### FIELD OF INVENTION

The present invention relates to use of ramipril in the manufacture of a medicament for the prevention of congestive heart failure (CHF), in patients with no preexisting CHF, where the patient is at high risk for a cardiovascular event due to a history of previous ischaemic heart disease, stroke or peripheral arterial disease and where the patient exhibits normal or low blood pressure.

### BACKGROUND OF THE INVENTION

Compounds that interfere with the renin angiotensin system (RAS) are well known in the art and are used to treat cardiovascular diseases, particularly arterial hypertension and heart failure. Principally, the RAS can be interferred with by inhibition of the enzymes synthesizing angiotensins or by blocking the corresponding receptors at the effector sites. Available today are inhibitors of the angiotensin converting enzyme (ACE) and angiotensin II type 1 receptor (AT II) antagonists.

ACE inhibitors are compounds which inhibit the conversion of angiotensin I into the active angiotensin II as well as the breakdown of the active vasodilator bradykinin. Both of these mechanisms lead to vasodilation. Such compounds have been described in, for example, EP 158927, EP 317878, US 4,743,450, and US 4,857,520.

Ramipril (disclosed in EP-A-079022) is a long-acting ACE inhibitor. Its active metabolite is the free diacid ramiprilat, which is obtained in vivo upon administration of ramipril. In hypertensive patients administration of ramipril is known to cause a reduction in peripheral arterial resistance and thus a reduction of the blood pressure without a compensatory rise in heart rate. It is currently being used in the treatment of hypertension and CHF. Furthermore, ramipril has been shown to reduce mortality in patients with clinical signs of congestive heart failure after surviving an acute myocardial infarction. Ramipril has been suggested to have an added advantage over many other ACE inhibitors due to its pronounced inhibition of ACE in tissues resulting in organ protective effects in e.g. the heart, kidney, and blood vessels.

Compounds that interfere with the RAS including ACE inhibitors and AT II antagonists are currently used in the treatment of various cardiovascular disorders, especially in patients exhibiting a high blood pressure. Use of said compounds in prevention of cardiovascular disorders is much less common and the use of said compounds in the prevention of CHF in patients with no preexisting CHF, where the patient is at high risk for a cardivascular event due to a history of previous ischaemic heart disease, stroke or peripheral arterial disease and where the patient exhibits normal or low blood pressure is hitherto unknown.

### SUMMARY OF THE INVENTION

The present invention relates to use of ramipril in the manufacture of a medicament for the prevention of development of CHF in patients with no preexisting CHF, i.e. no signs or symptoms of CHF, where the patient is at high risk for a cardiovascular event due to a history of previous ischaemic heart disease, stroke or peripheral aterial disease and where the patient exhibits normal or low blood pressure.

### DETAILED DESCRIPTION OF THE INVENTION

It has surprisingly been found that cardiovascular disorders such as CHF can be prevented by use of ramipril that interferes with the synthesis of angiotensin II. The present invention is especially surprising in that especially patients exhibiting a normal or low blood pressure benefit markedly from the preventive action of ramipril. The invention describes a new method to prevent CHF by administration of ramipril to patients with no preexisting CHF, where the patient is at high risk for a cardivascular event due to a history of previous ischaemic heart disease, stroke or peripheral arterial disease and where the patient exhibits normal or low blood pressure.

Patients exhibiting a normal or low blood pressure are known as normotensive patients. Examples of guidelines defining blood pressure values for different patient groups including different ages, include guidelines issued by the WHO and JNC (USA). In the present invention, a suitable definition of a normal or low blood pressure can be found in JNC VI, which is hereby incorporated by reference.

"Diabetes" include both type I diabetes, also known as insulin-dependent, diabetes mellitus (IDMM), and type II diabetes, also known as non-insulin-dependent diabetes mellitus (NIDDM).

In the present invention, ramipril is a compound which in itself or upon administration interferes with the synthesis of angiotensin II.

The present invention includes the mixture of isomers as well as the individual stereoisomers. The present invention further includes geometrical isomers, rotational isomers, enantiomers, racemates and diastereomers.

Where applicable, ramipril may be used in neutral form, e.g. as a carboxylic acid, or in the form of a salt, preferably a pharmaceutically acceptable salt such as the sodium, potassium, ammonium, calcium or magnesium salt of the compound at issue. Where applicable the compounds listed above can be used in hydrolyzable ester form.

In the present invention, ramipril may exhibit a long term duration, medium term duration or short term duration.

Ramipril or pharmaceutically acceptable salts thereof, including active metabolites, which can be used for the prevention of CHF.

Information about ramipril and ramiprilat can be obtained e.g. from the Merck Index., 12th ed., 1996, pp. 1394-1395.

For clinical use, ramipril is formulated into a pharmaceutical formulation for oral, intravenous, subcutaneous, tracheal, bronchial, intranasal, pulmonary, transdermal, buccal, rectal, parenteral or some other mode of administration. The pharmaceutical formulation may contain the inhibitor in admixture with a pharmaceutically acceptable adjuvant, diluent and/or carrier.

In the preparation of the pharmaceutical formulations of the present invention the active ingredient may be mixed with solid, powdered ingredients, such as lactose, saccharose, sorbitol, mannitol, starch, amylopectin, cellulose derivatives, gelatin, or another suitable ingredient, as well as with disintegrating agents and lubricating agents such as magnesium stearate, calcium stearate, sodium stearyl fumarate and polyethylene glycol waxes. The mixture may then be processed into granules or pressed into tablets.

The active ingredient may be separately premixed with the other, non-active ingredients, before being mixed to form a formulation.

Soft gelatine capsules may be prepared with capsules containing a mixture of the active ingredient of the invention, vegetable oil, fat, or other suitable vehicle for soft gelatine capsules. Hard gelatine capsules may contain granules of the active ingredients. Hard gelatine capsules may also contain the active ingredients in combination with solid powdered ingredients such as lactose, saccharose, sorbitol, mannitol, potato starch, corn starch, amylopectin, cellulose derivatives or gelatine.

Dosage units for rectal administration may be prepared (i) in the form of suppositories which contain the active substance mixed with a neutral fat base; (ii) in the form of a gelatine rectal capsule which contains the active substance in a mixture with a vegetable oil, paraffin oil or other suitable vehicle for gelatine rectal capsules; (iii) in the form of a ready-made micro enema; or (iv) in the form of a dry micro enema formulation to be reconstituted in a suitable solvent just prior to administration.

Liquid preparations may be prepared in the form of syrups or suspensions, e.g. solutions or suspensions containing the active ingredients and the remainder consisting, for example, of sugar or sugar alcohols and a mixture of ethanol, water, glycerol, propylene glycol and polyethylene glycol. If desired, such liquid preparations may contain coloring agents, flavoring agents, preservatives, saccharine and carboxymethyl cellulose or other thickening agents. Liquid preparations may also be prepared in the form of a dry powder to be recon-stituted with a suitable solvent prior to use.

Solutions for parenteral administration may be prepared as a solution of a formulation of the invention in a pharmaceutically acceptable solvent. These solutions may also contain stabilizing ingredients, preservatives and/or buffering ingredients. Solutions for parenteral administration may also be prepared as a dry preparation to by reconstituted with a suitable solvent before use.

The total amount of active ingredient suitably lies in the range of from about 0.1 % (w/w) to about 95 % (w/w) of the formulation, suitably from 0.5 % to 50 % (w/w) and preferably from 1 % to 25 % (w/w).

The pharmaceutical formulations may contain between about 0.1 mg and about 1000 mg of active ingredient, preferably between 1 mg and 100 mg of active ingredient.

The dose of the active ingredient to be administered will depend on the relevant indication, the age, weight and sex of the patient and may be determined by a physician. The dosage will suitably be in the range of from about 0.01 mg/kg to about 20 mg/kg, preferably between 0.1 mg/kg and 10 mg/kg.

The typical daily dose of the active ingredients varies within a wide range and will depend on various factors such as the relevant indication, the route of administration, the age, weight and sex of the patient and may be determined by a physician. In general, dosages, and especially oral and parenteral dosages, will be in the range of from about 0.1 to about 100 mg per day of active ingredient, preferably between 1 and 50 mg per day of active ingredient.

The following Example is intended to illustrate, but in no way limit the scope of the invention.

### EXAMPLE

A large-scale clinical trial was designed to examine the effect of the ACE inhibitor ramipril versus placebo in reducing cardiovascular events.

The study was conducted in 267 centres in 19 countries over a six year period and included 9,541 participants who are at high risk for cardiovascular events due to a history of previous ischaemic heart disease, stroke, peripheral arterial disease or individuals with diabetes.

The systolic blood pressure at inclusion of the patients was on average 138 mm Hg and thus the patients were normotensive at study start. After one month of therapy with either ramipril or placebo, the systolic blood pressure had decreased by 5.48 mm Hg and 1.59 mm Hg, respectively.

The primary endpoint of the study was myocardial infarction (MI), stroke and cardiovascular (CV) death (mortality).

The number of patients who developed CHF was significantly reduced by 21% in the ramipril group, which is unexpected since patients had no signs or symptoms of CHF at study start.

### Abbrevations

ACE = angiotensin converting enzyme
AT II = angiotensin II type 1 receptor
CHF = congestive heart failure
IDMM = insulin-dependent, diabetes mellitus
JNC = Joint National Committee
MI = myocardial infarction
NIDDM = non-insulin-dependent diabetes mellitus
WHO = World Health Organization

## Claims

1. The use of ramipril in the manufacture of a medicament for the prevention of congestive heart failure (CHF) in a patient with no preexisting CHF, where the patient is at high risk for a cardiovascular event due to a history of previous ischaemic heart disease, stroke or peripheral arterial disease, and where the patient exhibits normal or low blood pressure.

## Patentansprüche

1. Verwendung von Ramipril zur Herstellung eines Arzneimittels für die Verhinderung von Stauungsinsuffizienz (CHF) bei einem Patienten ohne vorher vorliegender CHF, wobei der Patient ein hohes Risiko für ein cardiovaskuläres Ereignis aufgrund einer Historie von früherer ischämischer Herzerkrankung, Schlaganfall oder peripherer arterieller Erkrankung aufweist und wobei der Patient einen normalen oder niedrigen Blutdruck zeigt.

## Revendications

1. Utilisation de ramipril dans la fabrication d'un médicament destiné à la prévention d'insuffisance cardiaque congestive (CHF) chez un patient sans CHF préexistante lorsque le patient présente un risque élevé d'accident cardio-vasculaire en raison d'antécédents de maladie cardiaque ischémique, d'accident vasculaire cérébral ou de maladie artérielle périphérique antérieurs, et lorsque le patient présente une tension artérielle normale ou basse.
